# EUROPÄISCHE PATENTSCHRIFT

(11) **EP 2 106 290 B1**
(45) Veröffentlichungstag und Bekanntmachung des Hinweises auf die Patenterteilung: **13.04.2016**
(21) Anmeldenummer: 07857473.8
(22) Anmeldetag: 12.12.2007
(51) Int. Cl.: B01J 8/06, B01J 8/04, B01J 35/00, B01J 35/02

(54) **KATALYSATORSYSTEM UND VERFAHREN ZUR GASPHASENOXIDATION UNTER VERWENDUNG EINER VORLAGE**
CATALYST SYSTEM AND METHOD FOR GAS PHASE OXIDATION USING AN UPSTREAM LAYER
SYSTÈME CATALYTIQUE ET PROCÉDÉ D'OXYDATION EN PHASE GAZEUSE EN UTILISANT UNE PRÉ-COUCHE

(30) Priorität: 21.12.2006 EP 06126893
(43) Veröffentlichungstag der Anmeldung: 07.10.2009
(73) Patentinhaber: BASF SE, 67056 Ludwigshafen am Rhein (DE)
(72) Erfinder: WILMER, Hagen, 67071 Ludwigshafen (DE); ZÜHLKE, Jürgen, 67346 Speyer (DE); LAUTENSACK, Thomas, 69488 Birkenau (DE); ALLMANN, Hans-Martin, 74867 Neunkirchen (DE); ROSOWSKI, Frank, 68239 Mannheim (DE); DOBNER, Cornelia, 67071 Ludwigshafen (DE)
(74) Vertreter: BASF IP Association
(86) Internationale Anmeldenummer: PCT/EP2007/063810
(87) Internationale Veröffentlichungsnummer: WO 2008/077791

(56) Entgegenhaltungen:
- EP-A- 1 108 470
- EP-A- 1 452 227
- WO-A-2006/092305
- DE-A1-102004 014 918
- GB-A- 721 412

## Beschreibung

Die vorliegende Erfindung betrifft ein Verfahren zur Gasphasenoxidation, bei dem man einen gasförmigen Strom, der einen aromatischen Kohlenwasserstoff und molekularen Sauerstoff umfasst, durch zwei oder mehrere Katalysatorlagen leitet. Ferner betrifft die vorliegende Erfindung ein Katalysatorsystem zur Gasphasenreaktion unter Verwendung einer Vorlage.

Eine Vielzahl von Carbonsäuren und/oder Carbonsäureanhydriden wird technisch durch die katalytische Gasphasenoxidation von aromatischen Kohlenwasserstoffen, wie Benzol, den Xylolen, Naphthalin, Toluol oder Durol, in Festbettreaktoren hergestellt. Man kann auf diese Weise z. B. Benzoesäure, Maleinsäureanhydrid, Phthalsäureanhydrid, Isophthalsäure, Terephthalsäure oder Pyromellithsäureanhydrid erhalten. Im Allgemeinen leitet man ein Gemisch aus einem sauerstoffhaltigen Gas und dem zu oxidierenden Ausgangsmaterial durch Rohre, in denen sich eine Schüttung eines Katalysators befindet. Zur Temperaturregelung sind die Rohre von einem Wärmeträgermedium, beispielsweise einer Salzschmelze, umgeben.

Obgleich die überschüssige Reaktionswärme durch das Wärmeträgermedium abgeführt wird, kann es in der Katalysatorschüttung zur Ausbildung lokaler Temperaturmaxima (Hot Spots) kommen, in denen eine höhere Temperatur herrscht als im übrigen Teil der Katalysatorschüttung. Diese Hot Spots führen zu Nebenreaktionen, wie der Totalverbrennung des Ausgangsmaterials, oder zur Bildung unerwünschter, vom Reaktionsprodukt nicht oder nur mit großem Aufwand abtrennbarer Nebenprodukte.

Außerdem kann der Katalysator ab einer bestimmten Hot Spot-Temperatur irreversibel geschädigt werden. Daher muss beim Anfahren des Verfahrens die Beladung des gasförmigen Stroms mit dem zu oxidierenden Kohlenwasserstoff anfangs sehr niedrig gehalten werden und kann nur langsam gesteigert werden. Der endgültige Produktionszustand wird oft erst nach einigen Wochen erreicht.

Seit den letzten Jahren wurden für die Oxidation von aromatischen Kohlenwasserstoffen Mehrlagenkatalysatorsysteme eingesetzt (beispielsweise DE-A 40 13 051, DE-A 198 23 262, EP-A 1 063 222, WO 2005/115616, EP-A 1 084 115, DE-A 103 23 818, DE-A 103 23 461, DE-A 103 23 817). Ziel dabei ist es, die Aktivität der einzelnen Katalysatorlagen dem Reaktionsverlauf entlang der Reaktorachse anzupassen. Dadurch ist es möglich, eine hohe Ausbeute an Wertprodukt und gleichzeitig eine möglichst geringe Ausbeute an den unerwünschten Zwischen- oder Nebenprodukten zu erzielen. Üblicherweise weisen die Katalysatoren der ersten, zum Reaktoreingang hin gelegenen Lage die geringste Aktivität auf, da im reaktoreingangsnahen Bereich die höchste Eduktkonzentration und damit die größten Reaktionsgeschwindigkeiten auftreten. Durch die bei der chemischen Reaktion frei werdende Wärme wird das Reaktionsgas aufgeheizt bis zu der Stelle, an der die durch Reaktion erzeugte Energie gerade so groß ist wie die an das Kühlmittel abgegebene Energie. Eine zu hohe Aktivität in der ersten Katalysatorlage würde zu einem unkontrollierten Ansteigen der Hot Spot-Temperatur führen, die üblicherweise zu einer Selektivitätsreduktion oder gar zu einem unkontrollierbaren Ablauf der Reaktion führen kann.

Ein weiterer Aspekt, der bei der Auslegung der Aktivität der einzelnen Katalysatorlagen beachtet werden muss, ist die Position des Hot Spots in der ersten Katalysatorlage. Da sich mit zunehmender Betriebszeit die Katalysatoraktivität verringert, gelangt ein höherer Anteil nicht umgesetzter Kohlenwasserstoffe oder teiloxidierter Intermediate in weiter stromabwärts gelegenen Bereiche der Katalysatorschüttung. Die Reaktion verlagert sich somit zunehmend zum Reaktorausgang und die Position des Hot Spots verschiebt sich immer weiter in Richtung Reaktorausgang. Dies kann sogar dahin führen, dass der Hot Spot aus der ersten Katalysatorlage in die zweite oder in eine später folgende Katalysatorlage wandert. Diese Wanderung des Hot Spots hat eine signifikant abfallende Wertproduktausbeute zur Folge.

Man kann der Katalysatordeaktivierung in begrenztem Umfang durch Erhöhung der Temperatur des Wärmeträgermediums entgegenwirken. Die Erhöhung der Temperatur des Wärmeträgermediums und/oder die Verschiebung des Hot Spots führen bei mehrlagigen Katalysatorsystemen dazu, dass die Temperatur, mit der das Gasgemisch in eine nachgelagerte Katalysatorlage eintritt, steigt. Da nachgelagerte Katalysatorlagen in der Regel zwar aktiver aber weniger selektiv sind, nehmen unerwünschte Überoxidation und andere Nebenreaktionen zu. Die zwei genannten Effekte bewirken, dass mit der Betriebsdauer die Produktausbeute bzw. Selektivität sinkt. In einem solchen Fall kann ein kompletter Katalysatoraustausch im Vergleich zum Weiterbetrieb wirtschaftlich sinnvoller wird.

Nachteilig an den im Stand der Technik angegebenen aktivitätsstrukturierten Katalysatorsystemen ist, dass trotz des Einsatzes solches strukturierten Katalysatorsysteme die Lebensdauer der Katalysatoren insbesondere in Hinblick auf die zunehmende Verlagerung des Hot Spots in die Richtung des Gasstroms nicht zufriedenstellend ist. Eine Positionierung des Hot Spots in einer aktiveren Katalysatorlage weiter zur Gasaustrittsseite hin beschränkt ferner die Möglichkeit der Feineinstellung der Selektivität des Katalysators zur Vermeidung von unerwünschten Nebenprodukten.

WO 2006/92305, WO 2006/92304 und WO 2007/116018 lösen dieses Problem durch die Verwendung einer aktiven Katalysatorlage, die den üblichen Katalysatorlagen zum Gaseintritt hin gelegen, vorgelagert ist. Die aktive Vorlage bewirkt ein schnelleres Aufheizen der Reaktorgase und somit einen früheren Start der chemischen Reaktion, so dass sich der Hot Spot im Vergleich zu den Systemen aus dem Stand der Technik weiter zum Gaseintritt hin ausbildet.

Ein Nachteil einer aktiveren Vorlage besteht allerdings darin, dass neben den Aktivmassensuspensionen für die nachfolgenden drei bis vier Katalysatorlagen eine weitere Aktivmassensuspension für die Vorlage zubereitet werden muss. Ferner bedeutet eine sehr aktive erste Lage am Reaktoreingang ein erhöhtes Sicherheitsrisiko, da die Reaktion wegen des hohen Eduktanteils am Reaktoreingang unkontrollierbar werden könnte.

Ein weiterer Nachteil des Stands der Technik ist, dass die Gastemperatur am Reaktoreingang weit unterhalb der Salzbadtemperatur liegt. In diesem Bereich dient die Salzbadtemperierung nicht der Abfuhr überschüssiger Wärme, sondern umgekehrt zum Aufwärmen des Reaktionsgases. Üblicherweise wird zum Aufheizen des Reaktionsgases unbefülltes, leeres Rohr, mit kugelförmigem Inertmaterial befülltes Rohr oder mit Katalysator befülltes Rohr verwendet. Der Nachteil von kugelförmigem Inertmaterial besteht in einem hohen Druckverlust über den Reaktor.

EP 1 452 227 offenbart Mischoxidkatalysatoren und ihre Verwendung zur Herstellung von Acrylsäure durch Gasphasenoxidation von Acrolein. DE 10 2004 014 918 A1 beschreibt ringförmige Katalysatoren mit einer Silber-Vanadiumoxidphase und einer Promotorphase, welche zur Herstellung von Carbonsäureanhydriden verwendet werden können. EP 1 108 470 offenbart ringförmige Trägerkatalysatoren zur Verwendung in Gasphasenoxidationen von Kohlenwasserstoffen, welche Kerben an den Flachseiten der Ringe besitzen.

Die Aufgabe der vorliegenden Erfindung bestand danach darin, ein Verfahren zur Gasphasenoxidation von aromatischen Kohlenwasserstoffen und ein Katalysatorsystem zur Durchführung dieses Verfahrens bereitzustellen, in dem die Aufheizung der Reaktionsgase am Reaktoreingang schneller als im Stand der Technik erfolgen, bzw. in dem die Aufheizung der Reaktionsgase am Reaktoreingang in einer vergleichbaren Zeit wie unter Verwendung der Erfindung von WO 2006/92305, WO 2006/92304 oder WO 2007/116018 erfolgt, wobei allerdings das Erreichen dieses schnelleren Aufheizens einfacher durchzuführen ist und kein erhöhtes Sicherheitsrisiko mit sich bringt. Durch eine schnellere Aufheizung kann ein schnelleres "Anspringen" der Reaktion erreicht werden, wobei sich der Hot Spot weiter zum Gaseintritt hin gelegen ausbildet. Eine weitere Aufgabe bestand demnach darin, ein Katalysatorsystem aufzuzeigen, dessen Hot Spot sich weiter zum Gaseintritt hin gelegen ausbildet als im Stand der Technik. Folglich sollen Katalysatorsysteme aufgezeigt werden, die eine längere Standzeit auf einem hohen Ausbeuteniveau erreichen.

Die Aufgabe wurde gelöst durch ein Verfahren zur Gasphasenoxidation, bei dem man einen gasförmigen Strom, der wenigstens einen aromatischen Kohlenwasserstoff und molekularen Sauerstoff umfasst, durch wenigstens zwei in Strömungsrichtung des gasförmigen Stroms hintereinander angeordnete Katalysatorlagen aus Katalysator-Ringen leitet, wobei die Aktivität der Katalysatoren in aneinandergrenzenden Katalysatorlagen voneinander verschieden ist, das dadurch gekennzeichnet ist, dass den aneinandergrenzenden Katalysatorlagen entgegengesetzt zur Strömungsrichtung des Gases eine Katalysator- und/oder Inertlage vorgelagert ist,
wobei das Volumen der vorgelagerten Inert- und/oder Katalysator-Ringe kleiner ist als bei mindestens einer der folgenden Katalysatorlagen oder
wobei der Quotient aus Oberfläche pro Volumen der vorgelagerten Inert- und/oder Katalysator-Ringe größer ist als bei mindestens einer der folgenden Katalysatorlagen und das Volumen durch Einkerbungen an jeder Stirnseite der Ringe verringert wird.

Die vorgelagerte Katalysator- und/oder Inertlage stellt demnach die erste Lage (zum Gaseintritt hin gelegen) des Katalysatorsystems dar.

In der Vorlage können auch Mischungen aus verschiedenen Inert- und/oder Katalysator-Ringen vorliegen. Die genannten Angaben der vorgelagerten Inert- und/oder Katalysator-Ringe beziehen sich dann jeweils auf das mittlere Volumen oder den mittleren Quotienten.

Bei dem Begriff "Durchmesser" ist im Rahmen dieser Erfindung der mittlere äußere Durchmesser der vorgelagerten Inert- und/oder Katalysator-Ringe gemeint.

Bei dem Begriff "Volumen" ist im Rahmen dieser Erfindung das geometrische Volumen des Rings gemeint.

Vorteilhaft ist das Volumen der vorgelagerten Inert- und/oder Katalysator-Ringe kleiner als das von mindestens zwei, bevorzugt mindestens drei, insbesondere aller der folgenden Katalysatorlagen.

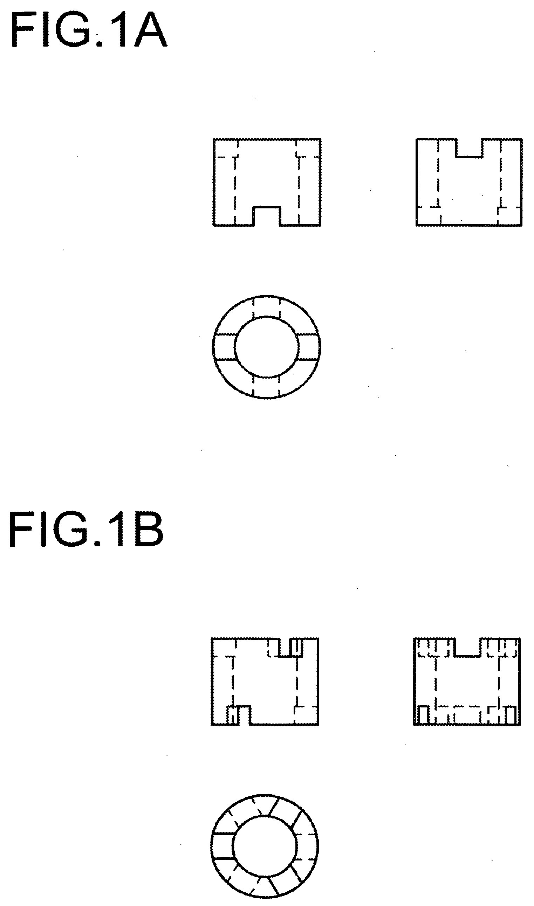

Das Volumen der vorgelagerten Katalysator-Ringe kann beispielsweise durch Einkerbungen oder Schlitze, die sich über den kompletten Katalysatorring erstrecken, verringert werden. Besonders bevorzugt wird das Volumen durch mehrere Einkerbungen an der Oberseite und mehrere Einkerbungen an der Unterseite der Ringe (an jeder Stirnseite) verringert. Ganz besonders bevorzugt wird das Volumen durch zwei, drei, vier oder fünf symmetrisch angeordnete Einkerbungen an der Oberseite und zwei, drei, vier oder fünf symmetrisch angeordnete Einkerbungen an der Unterseite der Ringe (an jeder Stirnseite) verringert. Weiterhin ganz besonders bevorzugt wird das Volumen durch zwei symmetrische Einkerbungen an der Oberseite und zwei symmetrische Einkerbungen an der Unterseite der Ringe (an jeder Stirnseite) verringert, wobei die Einkerbungen der Oberseite und die der Unterseite identisch sind, nur um 90° gedreht. Diese Einkerbungen werden in der Figur 1 a verdeutlicht. Weiterhin ganz besonders bevorzugt wird das Volumen durch drei symmetrische Einkerbungen an der Oberseite und drei symmetrische Einkerbungen an der Unterseite der Ringe (an jeder Stirnseite) verringert, wobei die Einkerbungen der Oberseite und die der Unterseite identisch sind. Diese Einkerbungen werden in der Figur 1 b verdeutlicht.

Vorteilhaft ist der Quotient aus Oberfläche pro Volumen der vorgelagerten Inert- und/oder Katalysator-Ringe größer als der von mindestens zwei, bevorzugt mindestens drei, insbesondere aller der folgenden Katalysatorlagen.

Vorteilhaft folgen der vorgelagerten Inert- und/oder Katalysatorlage mindestens drei weitere Katalysatorlagen, bevorzugt 3 bis 5 weitere Katalysatorlagen.

Als Katalysatorlage wird vorliegend die Schüttung eines Katalysators mit im Wesentlichen einheitlicher Aktivität angesehen, d. h. mit im Wesentlichen einheitlicher Zusammensetzung der Aktivmasse, Aktivmasseanteil und Packungsdichte (abgesehen von unvermeidlichen Fluktuationen beim Befüllen des Reaktors). Aufeinanderfolgende Katalysatorlagen unterscheiden sich somit in der Aktivität der enthaltenen Katalysatoren. Dem Fachmann sind verschiedene Maßnahmen zur Steuerung der Katalysatoraktivität bekannt.

Vorteilhaft nimmt die Aktivität der Katalysatorlagen von der ersten Katalysatorlage nach der vorgelagerten Inert- und/oder Katalysatorlage kontinuierlich bis zur letzten Katalysatorlage (in Strömungsrichtung gesehen) zu. Es können aber auch Zwischenlagen aus Katalysatoren mit geringer Aktivität oder aus Inertmaterial integriert werden (siehe beispielsweise WO 2007/125096).

Vorteilhaft beträgt der Durchmesser der vorgelagerten Inert- oder Katalysator-Ringe 3 bis 8 mm, bevorzugt 5 bis 7 mm. Vorteilhaft beträgt die Höhe der vorgelagerten Inert- oder Katalysator-Ringe 3 bis 8 mm, bevorzugt 5 bis 7 mm. Vorteilhaft beträgt der innere Durchmesser der vorgelagerten Inert- oder Katalysator-Ringe 1 bis 6 mm, bevorzugt 3 bis 5 mm.

Vorteilhaft ist das Volumen der vorgelagerten Inert- oder Katalysator-Ringe um mindestens 5 bis 10 % kleiner als das Volumen mindestens einer der nachfolgenden Katalysatorlagen. Vorteilhaft ist das Volumen um mindestens 10 bis 20 % kleiner. Vorteilhaft liegt das Volumen der vorgelagerten Inert- oder Katalysator-Ringe bei kleiner gleich 170 mm³, bevorzugt bei kleiner gleich 110 mm³. Das Volumen der vorgelagerten Inert- oder Katalysator-Ringe liegt vorteilhaft zwischen 25 bis 170 mm³, bevorzugt zwischen 60 und 110 mm³.

Vorteilhaft ist der Quotient aus Oberfläche pro Volumen der vorgelagerten Inert- oder Katalysator-Ringe um mindestens 5 bis 10 % größer als der Quotient aus Oberfläche pro Volumen mindestens einer der nachfolgenden Katalysatorlagen. Vorteilhaft ist der Quotient aus Oberfläche pro Volumen um mindestens 10 bis 20 % größer. Vorteilhaft liegt der Quotient aus Oberfläche pro Volumen der vorgelagerten Inert- oder Katalysator-Ringe bei größer gleich 180 mm⁻¹, bevorzugt bei größer gleich 200 mm¹. Der Quotient aus Oberfläche pro Volumen der vorgelagerten Inert- oder Katalysator-Ringe liegt vorteilhaft zwischen 180 bis 500 mm⁻¹, bevorzugt zwischen 200 und 300 mm⁻¹.

Vorteilhaft weisen die vorgelagerten Inert- und/oder Katalysator-Ringe keine Aktivität bis zur Aktivität der ersten nachfolgenden Katalysatorlage auf. Besonders bevorzugt weisen die vorgelagerten Inert- und/oder Katalysator-Ringe entweder keine Aktivität oder die Aktivität der ersten nachfolgenden Katalysatorlage auf.

Unter Aktivität eines Katalysators bzw. einer Katalysatorlage wird der Umsatz verstanden, der unter identischen Bedingungen (insbesondere hinsichtlich Katalysatorvolumen, volumenbezogener Raumgeschwindigkeit (gas hourly space velocity GHSV) bzw. Luftmenge, Temperatur des Wärmeträgermediums, Kohlenwasserstoff-Beladung des gasförmigen Stroms) in einer Testanlage gemessen wird. Je höher der Umsatz eines Katalysators bzw. einer Katalysatorlage desto höher ist dessen/deren Aktivität. Diese Methode ist insbesondere zum Vergleich von Aktivitäten bzw. zur Bestimmung relativer Katalysatoraktivitäten geeignet.

Dem Fachmann sind die Methoden zur Steuerung der Aktivität einer Katalysatorlage hinlänglich bekannt. Beispielsweise kann die Aktivität/Selektivität der Katalysatoren durch Zugabe von Promotoren in die Aktivmasse, durch Einstellung der BET-Oberfläche der Katalysatoren, durch den Aktivmassenanteil, d.h. durch die Aktivmasse pro Rohrvolumen, durch den Leerraum zwischen den einzelnen Katalysatorformkörpern oder durch den Gehalt an Inertstoffen variiert werden.

Im Fall einer inaktiven Vorlage besteht diese aus einem inerten Material, wie es z. B. auch als Katalysatorträger verwendet wird. Geeignete Trägermaterialien sind beispielsweise Quarz (SiO₂), Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Die inaktive Vorlagelage kann auch Gewebe, Gestricke oder Gewirke aus Fasern oder Metalldrähten umfassen.

Die Gesamtlänge aller Katalysatorlagen inklusive der Vorlage aus Inert- und/oder Katalysatormaterial beträgt üblicherweise 2,5 bis 4 m, bevorzugt 2,8 bis 3,4 m. Die Länge der Vorlage beträgt vorteilhaft 0,05 bis 1 m, bevorzugt 0,1 bis 0,5 m, insbesondere 0,15 bis 0,4 m. Demnach beträgt die Schüttungslänge der vorgelagerten Inert- und/oder Katalysator-Ringe vorteilhaft 1,5 bis 40 % der Gesamtschüttungslänge, bevorzugt 2,5 bis 20%, insbesondere 3,5 bis 15 %

Vorteilhaft weist in einem Dreilagen-Katalysatorsystem mit vorgelagerter Inert- und/oder Katalysatorlage, bezogen auf die Gesamtlänge des Katalysatorbettes, die Inert- und/oder Katalysatorlage eine Schüttungslänge von 1 bis 40 %, bevorzugt 5 bis 25 %, insbesondere 10 bis 20 %, auf. Die erste nachfolgende Katalysatorlage weist vorteilhaft, bezogen auf die Gesamtlänge des Katalysatorbettes, eine Schüttungslänge von 15 bis 75 %, bevorzugt 25 bis 60 %, insbesondere 30 bis 50 %, auf. Die zweite nachfolgende Katalysatorlage weist vorteilhaft, bezogen auf die Gesamtlänge des Katalysatorbettes, eine Schüttungslänge von 5 bis 45 %, bevorzugt 10 bis 40 %, insbesondere 15 bis 30 %, auf. Die dritte nachfolgende Katalysatorlage weist ebenso vorteilhaft, bezogen auf die Gesamtlänge des Katalysatorbettes, eine Schüttungslänge von 5 bis 45 %, bevorzugt 10 bis 40 %, insbesondere 15 bis 30 %, auf.

Vorteilhaft beträgt in einem Dreilagen-Katalysatorsystem mit vorgelagerter Inert- und/oder Katalysatorlage die Schüttungslänge der vorgelagerten Katalysatorlage 5 cm bis 120 cm, bevorzugt 15 cm bis 75 cm, insbesondere 30 cm bis 60 cm, die Schüttungslänge der ersten nachfolgenden Katalysatorlage 45 cm bis 225 cm, bevorzugt 75 cm bis 180 cm, insbesondere 90 cm bis 150 cm, die Schüttungslänge der zweiten nachfolgenden Katalysatorlage 15 cm bis 135 cm, bevorzugt 30 cm bis 120 cm, insbesondere 45 cm bis 90 cm und die Schüttungslänge der dritten nachfolgenden Katalysatorlage 15 cm bis 135 cm, bevorzugt 30 cm bis 120 cm, insbesondere 45 cm bis 90 cm.

Die katalytische Zusammensetzung eines Dreilagen-Katalysatorsystems ist beispielsweise in der WO 2004/103561 auf der Seite 7 beschrieben.

Vorteilhaft weist in einem Vierlagen-Katalysatorsystem mit vorgelagerter Inert- und/oder Katalysatorlage die vorgelagerte Inert- und/oder Katalysatorlage, bezogen auf die Gesamtlänge des Katalysatorbettes, eine Schüttungslänge von 1 bis 40 %, bevorzugt 5 bis 25 %, insbesondere 10 bis 20 %, auf. Die erste nachfolgende Katalysatorlage weist vorteilhaft, bezogen auf die Gesamtlänge des Katalysatorbettes, eine Schüttungslänge von 15 bis 75 %, bevorzugt 25 bis 60 %, insbesondere 30 bis 50 %, auf. Die zweite nachfolgende Katalysatorlage weist vorteilhaft, bezogen auf die Gesamtlänge des Katalysatorbettes, eine Schüttungslänge von 5 bis 45 %, bevorzugt 5 bis 30 %, insbesondere 10 bis 20 %, auf. Die dritte nachfolgende Katalysatorlage weist vorteilhaft, bezogen auf die Gesamtlänge des Katalysatorbettes, eine Schüttungslänge von 5 bis 45 %, bevorzugt 5 bis 30 %, insbesondere 10 bis 25 %, auf. Die vierte nachfolgende Katalysatorlage weist ebenso vorteilhaft, bezogen auf die Gesamtlänge des Katalysatorbettes, eine Schüttungslänge von 5 bis 45 %, bevorzugt 5 bis 30 %, insbesondere 10 bis 25 %, auf.

Vorteilhaft beträgt in einem Vierlagen-Katalysatorsystem mit vorgelagerter Inert- und/oder Katalysatorlage die Schüttungslänge der vorgelagerten Inert- und/oder Katalysatorlage 5 cm bis 120 cm, bevorzugt 15 cm bis 75 cm, insbesondere 30 cm bis 60 cm, die Schüttungslänge der ersten nachfolgenden Katalysatorlage 45 cm bis 225 cm, bevorzugt 75 cm bis 180 cm, insbesondere 90 cm bis 150 cm, die Schüttungslänge der zweiten nachfolgenden Katalysatorlage 15 cm bis 135 cm, bevorzugt 15 cm bis 90 cm, insbesondere 30 cm bis 60 cm, die Schüttungslänge der dritten nachfolgenden Katalysatorlage 15 cm bis 135 cm, bevorzugt 15 cm bis 90 cm, insbesondere 30 cm bis 75 cm und die Schüttungslänge der vierten nachfolgenden Katalysatorlage 15 cm bis 135 cm, bevorzugt 15 cm bis 90 cm, insbesondere 30 cm bis 75 cm.

Die katalytische Zusammensetzung eines Vierlagen-Katalysatorsystems ist beispielsweise in der WO 2004/103561 auf der Seite 8 oder in der WO 2007/135002 auf der Seite auf den Seite 5 und 6 beschrieben.

Vorteilhaft bilden sich in der vorgelagerten Katalysatorlage keine Hot Spots aus.

Besonders bevorzugt teilt sich die Vorlage in zwei Bereiche auf. Der erste Bereich, zum Gaseintritt hin gelegen, weist eine Schüttung aus Inertmaterial auf, während der zweite Bereich, in Strömungsrichtung folgend (zu den anderen Katalysatorlagen hin gelegen) eine Schüttung aus Katalysatormaterial enthält,
wobei das Volumen der vorgelagerten Inert- und Katalysator-Ringe kleiner ist als bei mindestens einer der folgenden Katalysatorlagen oder
wobei der Quotient aus Oberfläche pro Volumen der vorgelagerten Inert- und Katalysator-Ringe größer ist als bei mindestens einer der folgenden Katalysatorlagen und das Volumen durch Einkerbungen an jeder Stirnseite der Ringe verringert wird.

Vorteilhaft nimmt der erste Bereich der Inertlage der Vorlage 5 bis 25 cm ein, bevorzugt 10 bis 20 cm. Der zweite Bereich der Katalysatormaterialschüttung der Vorlage nimmt vorteilhaft 10 bis 75 cm ein, bevorzugt 30 bis 50 cm. Demnach nimmt der erste Bereich der Inertlage der Vorlage vorteilhaft 5 bis 70 % der gesamten Vorlage ein, bevorzugt 15 bis 40 % ein. Der zweite Bereich der Katalysatormaterialschüttung der Vorlage nimmt folglich vorteilhaft 30 bis 95 % der gesamten Vorlage ein, bevorzugt 60 bis 85 %. Die Aktivität der vorgelagerten Katalysatorlage entspricht vorteilhaft der Aktivität der ersten nachfolgenden Katalysatorlage.

Neben der Vorlage kann gegebenenfalls zum Druckverlustabgleich über die einzelnen Rohre des Rohrbündelreaktors weiteres Inert- und/oder Katalysatormaterial eingesetzt werden.

Das erfindungsgemäße Verfahren eignet sich besonders zur Gasphasenoxidation aromatischer C₆- bis C₁₀-Kohlenwasserstoffe, wie Benzol, den Xylolen, Toluol, Naphthalin oder Durol (1,2,4,5-Tetramethylbenzol) zu Carbonsäuren und/oder Carbonsäureanhydriden wie Maleinsäureanhydrid, Phthalsäureanhydrid, Benzoesäure und/oder Pyromellithsäuredianhyrid. Das Verfahren eignet sich besonders zur Herstellung von Phthalsäureanhydrid aus o-Xylol und/oder Naphthalin.

Die Durchführung des erfindungsgemäßen Verfahrens ist dem Fachmann bekannt und beispielsweise in der in der WO 2004/103561 auf den Seiten 6 und 7 beschrieben.

Das erfindungsgemäße Verfahren bewirkt eine schnelle Aufheizung des Reaktionsgases am Anfang der Katalysatorschüttung.

Im Gegensatz zur WO 2006/92305, WO 2006/92304 und WO 2007/116018 wird in der vorliegenden Erfindung das Reaktionsgas zwar ebenso unterhalb der Salzbadtemperatur aufgeheizt, aber es ergibt sich kein erhöhtes Sicherheitsrisiko, wenn die Gastemperatur in der Vorlage die Salzbadtemperatur überschreiten sollte.

Ferner betrifft die vorliegende Erfindung ein Katalysatorsystem zur Durchführung von Verfahren zur Gasphasenoxidation, bei dem man einen gasförmigen Strom, der wenigstens einen aromatischen Kohlenwasserstoff und molekularen Sauerstoff umfasst, mit der Maßgabe, dass das Katalysatorsystem wenigstens zwei in Strömungsrichtung des gasförmigen Stroms hintereinander angeordnete Katalysatorlagen aus Katalysator-Ringen aufweist, wobei die Aktivität der Katalysatoren in aneinandergrenzenden Katalysatorlagen voneinander verschieden ist, mit der Maßgabe, dass den aneinandergrenzenden Katalysatorlagen entgegengesetzt zur Strömungsrichtung des Gases eine Katalysator- und/oder Inertlage vorgelagert ist,
wobei das Volumen der vorgelagerten Inert- und/oder Katalysator-Ringe kleiner ist als bei mindestens einer der folgenden Katalysatorlagen oder
wobei der Quotient aus Oberfläche pro Volumen der vorgelagerten Inert- und/oder Katalysator-Ringe größer ist als bei mindestens einer der folgenden Katalysatorlagen und das Volumen durch Einkerbungen an jeder Stirnseite der Ringe verringert wird.

Durch die vorliegende Erfindung wird ein Katalysatorsystem zur Verfügung gestellt, dessen anfänglicher Hot Spot sich sehr nahe am Reaktoreingang ausbildet. Durch die stärkere Nutzung der zum Reaktoreingang hin gelegenen Katalysatorschüttung können längere Standzeiten erreicht werden. Ferner treten die genannten unerwünschten Nebenreaktionen durch das Wandern des Hot Spots in aktivere Katalysatorlagen erst zu einem späteren Zeitpunkt auf als bei Katalysatorsysteme aus dem Stand der Technik.

Die Erfindung wird durch die beigefügten Figuren 1a und 1b und die folgenden Beispiele näher veranschaulicht.

### Beispiele

Katalysator 1: gemäß US 6586361, Beispiel 9 (d.h. "catalyst lie"),Reihe 8, mit einer Geometrie von 8 x 6 x 5 mm (äußerer Durchmesser x Höhe x innerer Durchmesser) Katalysator 2: gemäß US 6586361, Beispiel 10 (d.h. "catalyst III"), Reihe 8, mit einer Geometrie von 8 x 6 x 5 mm (äußerer Durchmesser x Höhe x innerer Durchmesser)

### Vorlagen:

Vorlage 1: Inerte Steatitringe mit einer Geometrie von 5 x 3 x 2 mm (äußerer Durchmesser x Höhe x innerer Durchmesser)
Vorlage 2: Inerte Steatitringe mit einer Geometrie von 7 x 7 x 4 mm (äußerer Durchmesser x Höhe x innerer Durchmesser), die auf jeder Stirnseite gemäß Figur 1a Einkerbungen mit einem Einschnitt von 1,5 mm x 1,5 mm aufweisen.
Vorlage 3: 10,1 % Aktivmasse auf Steatitringen, wobei die Zusammensetzung der von Katalysator 1 entspricht, mit einer Geometrie von 7 x 7 x 4 mm (äußerer Durchmesser x Höhe x innerer Durchmesser)
Vorlage 4: Inerte Steatitringe mit einer Geometrie von 8 x 6 x 5 mm (äußerer Durchmesser x Höhe x innerer Durchmesser)

### Katalysatorsystem-Test:

Test 1: nicht erfindungsgemäß, Katalysatorsystem in Strömungsrichtung: 10 cm Vorlage 1, 170 cm Katalysator 1, 130 cm Katalysator 2
Test 2: erfindungsgemäß, Katalysatorsystem in Strömungsrichtung: 10 cm Vorlage 2, 170 cm Katalysator 1, 130 cm Katalysator 2
Test 3: erfindungsgemäß, Katalysatorsystem in Strömungsrichtung: 10 cm Vorlage 2, 40 cm Vorlage 3, 170 cm Katalysator 1, 130 cm Katalysator 2
Test 4: nicht erfindungsgemäß, Katalysatorsystem in Strömungsrichtung: 10 cm Vorlage 4, 170 cm Katalysator 1, 130 cm Katalysator 2

### Durchführung der Katalysatorsystem-Tests:

Die vier Katalysatorsysteme wurden bei einer Beladung von 70 g/Nm3 o-Xylol getestet.
Bei gleicher Salzbadtemperatur von 355°C wurden folgende in Tabelle 1 zusammengestellte Hot Spot Positionen gemessen.

**Tabelle 1:**

| Test | Hot Spot Position [cm] |
|---|---|
| 1 | 65 |
| 2 | 80 |
| 3 | 75 |
| 4 | 90 |

## Patentansprüche

1. Verfahren zur Gasphasenoxidation, bei dem man einen gasförmigen Strom, der wenigstens einen aromatischen Kohlenwasserstoff und molekularen Sauerstoff umfasst, durch wenigstens zwei in Strömungsrichtung des gasförmigen Stroms hintereinander angeordnete Katalysatorlagen aus Katalysator-Ringen leitet, wobei die Aktivität der Katalysatoren in aneinandergrenzenden Katalysatorlagen voneinander verschieden ist, **dadurch gekennzeichnet, dass** den aneinandergrenzenden Katalysatorlagen entgegengesetzt zur Strömungsrichtung des Gases eine Katalysator- und/oder Inertlage vorgelagert ist,
wobei das Volumen der vorgelagerten Inert- oder Katalysator-Ringe kleiner ist als bei mindestens einer der folgenden Katalysatorlagen oder
wobei der Quotient aus Oberfläche pro Volumen der vorgelagerten Inert- oder Katalysator-Ringe größer ist als bei mindestens einer der folgenden Katalysatorlagen und das Volumen durch Einkerbungen an jeder Stirnseite der Ringe verringert wird.

2. Verfahren nach Anspruch 1, **dadurch gekennzeichnet, dass** das Volumen der vorgelagerten Inert- oder Katalysator-Ringe um mindestens 5 bis 10 % kleiner ist als bei mindestens einer der folgenden Katalysatorlagen oder der Quotient aus Oberfläche pro Volumen der vorgelagerten Inert- oder Katalysator-Ringe mindestens 5 bis 10 % größer ist als bei mindestens einer der folgenden Katalysatorlagen.

3. Verfahren nach Anspruch 1 oder 2, **dadurch gekennzeichnet, dass** das Volumen der vorgelagerten Inert- oder Katalysator-Ringe kleiner gleich 170 mm³ ist oder der Quotient aus Oberfläche pro Volumen der vorgelagerten Inert- oder Katalysator-Ringe größer gleich 180 mm⁻¹ ist.

4. Verfahren nach den Ansprüchen 1 bis 3, **dadurch gekennzeichnet, dass** die Schüttungslänge der vorgelagerten Inert- und Katalysator-Ringe 2,5 bis 20 % der Gesamtschüttungslänge beträgt.

5. Verfahren nach den Ansprüchen 1 bis 4, **dadurch gekennzeichnet, dass** die vorgelagerte Inert- oder Katalysatorlage keine Aktivität oder die Aktivität der ersten nachfolgenden Katalysatorlage aufweist.

6. Verfahren nach den Ansprüchen 1 bis 5, **dadurch gekennzeichnet, dass** die vorgelagerte Katalysator- und/oder Inertlage einen Bereich aus Inertmaterial und einen Bereich aus Katalysatormaterial beinhaltet, wobei der Bereich aus Inertmaterial zum Gaseintritt hin gelegen ist.

7. Verfahren nach den Ansprüchen 1 bis 6, **dadurch gekennzeichnet, dass** der Inertmaterialbereich 15 bis 40 % der Gesamtlänge der Vorlage ausmacht.

8. Verfahren nach den Ansprüchen 1 bis 7, **dadurch gekennzeichnet, dass** Phthalsäureanhydrid aus o-Xylol und/oder Naphthalin hergestellt wird.

9. Katalysatorsystem zur Durchführung von Verfahren zur Gasphasenoxidation, bei dem man einen gasförmigen Strom, der wenigstens einen aromatischen Kohlenwasserstoff und molekularen Sauerstoff umfasst, mit der Maßgabe, dass das Katalysatorsystem wenigstens zwei in Strömungsrichtung des gasförmigen Stroms hintereinander angeordnete Katalysatorlagen aus Katalysator-Ringen aufweist, wobei die Aktivität der Katalysatoren in aneinandergrenzenden Katalysatorlagen voneinander verschieden ist, mit der Maßgabe, dass den aneinandergrenzenden Katalysatorlagen entgegengesetzt zur Strömungsrichtung des Gases eine Katalysator- und/oder Inertlage vorgelagert ist,
wobei das Volumen der vorgelagerten Inert- und/oder Katalysator-Ringe kleiner ist als bei mindestens einer der folgenden Katalysatorlagen oder
wobei der Quotient aus Oberfläche pro Volumen der vorgelagerten Inert- und/oder Katalysator-Ringe größer ist als bei mindestens einer der folgenden Katalysatorlagen und das Volumen durch Einkerbungen an jeder Stirnseite der Ringe verringert wird.

## Claims

1. A process for gas-phase oxidation, in which a gaseous stream comprising at least one aromatic hydrocarbon and molecular oxygen is passed through at least two catalyst zones consisting of catalyst rings and arranged in series in the flow direction of the gaseous stream and the activities of the catalysts in adjoining catalyst zones are different from one another, wherein a catalyst and/or inert zone precedes the adjoining catalyst zones in a direction opposite to the flow direction of the gas, with the volume of the preceding inert or catalyst rings being smaller than in the case of at least one of the subsequent catalyst zones or the ratio of surface area to volume of the preceding inert or catalyst rings being greater than in the case of at least one of the subsequent catalyst zones and the volume being reduced by means of notches on each end face of the rings.

2. The process according to claim 1, wherein the volume of the preceding inert or catalyst rings is at least 5-10% smaller than in the case of at least one of the subsequent catalyst zones or the ratio of surface area to volume of the preceding inert or catalyst rings is at least 5-10% greater than in the case of at least one of the subsequent catalyst zones.

3. The process according to claim 1 or 2, wherein the volume of the preceding inert or catalyst rings is less than or equal to 170 mm³ or the ratio of surface area to volume of the preceding inert or catalyst rings is greater than or equal to 180 mm¹.

4. The process according to any of claims 1 to 3, wherein the bed length of the preceding inert- and/or catalyst rings is from 2.5 to 20% of the total bed length.

5. The process according to any of claims 1 to 4, wherein the preceding inert or catalyst zone has no activity or the activity of the first subsequent catalyst zone.

6. The process according to any claims of 1 to 5, wherein the preceding catalyst and/or inert zone comprises a region composed of inert material and a region composed of catalyst material, with the region composed of inert material being closest to the gas inlet.

7. The process according to any of claims 1 to 6, wherein the inert material region makes up from 15 to 40% of the total length of the preliminary zone.

8. The process according to any of claims 1 to 7, wherein phthalic anhydride is prepared from oxylene and/or naphthalene.

9. A catalyst system for carrying out gas-phase oxidation processes, in which a gaseous stream comprising at least one aromatic hydrocarbon and molecular oxygen, with the proviso that the catalyst system comprises at least two catalyst zones consisting of catalyst rings and arranged in series in the flow direction of the gaseous stream and the activities of the catalysts in adjoining catalyst zones are different from one another, with the proviso that a catalyst and/or inert zone precedes the adjoining catalyst zones in a direction opposite to the flow direction of the gas, with the volume of the preceding inert and/or catalyst rings being smaller than in the case of at least one of the subsequent catalyst zones or the ratio of surface area to volume of the preceding inert and/or catalyst rings being greater than in the case of at least one of the subsequent catalyst zones and the volume being reduced by means of notches on each end face of the rings.

## Revendications

1. Procédé d'oxydation en phase gazeuse, selon lequel un courant gazeux, qui comprend au moins un hydrocarbure aromatique et de l'oxygène moléculaire, est conduit au travers d'au moins deux couches de catalyseur constituées d'anneaux de catalyseur, agencées successivement dans la direction d'écoulement du courant gazeux, l'activité des catalyseurs dans des couches de catalyseur adjacentes étant différente les unes des autres, **caractérisé en ce qu'**une couche de catalyseur et/ou inerte est disposée en amont des couches de catalyseurs adjacentes dans la direction opposée à la direction d'écoulement du gaz,
le volume des anneaux inertes ou de catalyseur en amont étant inférieur à celui dans au moins une des couches de catalyseur suivantes, ou
le quotient de la surface par volume des anneaux inertes ou de catalyseur en amont étant supérieur à celui dans au moins une des couches de catalyseur suivantes et le volume étant réduit par des encoches sur chaque face des anneaux.

2. Procédé selon la revendication 1, **caractérisé en ce que** le volume des anneaux inertes ou de catalyseur en amont est au moins 5 à 10 % plus petit que celui dans au moins une des couches de catalyseur suivantes, ou le quotient de la surface par volume des anneaux inertes ou de catalyseur en amont est au moins 5 à 10 % plus grand que celui dans au moins une des couches de catalyseur suivantes.

3. Procédé selon la revendication 1 ou 2, **caractérisé en ce que** le volume des anneaux inertes ou de catalyseur en amont est inférieur ou égal à 170 mm³ ou le quotient de la surface par volume des anneaux inertes ou de catalyseur en amont est supérieur ou égal à 180 mm⁻¹.

4. Procédé selon les revendications 1 à 3, **caractérisé en ce que** la longueur de garnissage des anneaux inertes et de catalyseur en amont est de 2,5 à 20 % de la longueur de garnissage totale.

5. Procédé selon les revendications 1 à 4, **caractérisé en ce que** la couche inerte ou de catalyseur en amont ne présente aucune activité ou présente l'activité de la première couche de catalyseur suivante.

6. Procédé selon les revendications 1 à 5, **caractérisé en ce que** la couche de catalyseur et/ou inerte en amont contient une zone de matériau inerte et une zone de matériau catalytique, la zone de matériau inerte étant disposée vers l'entrée du gaz.

7. Procédé selon les revendications 1 à 6, **caractérisé en ce que** la zone de matériau inerte représente 15 à 40 % de la longueur totale de la couche en amont.

8. Procédé selon les revendications 1 à 7, **caractérisé en ce que** de l'anhydride de l'acide phtalique est fabriqué à partir d'o-xylène et/ou de naphtaline.

9. Système catalytique pour la réalisation de procédés d'oxydation en phase gazeuse, selon lequel un courant gazeux, qui comprend au moins un hydrocarbure aromatique et de l'oxygène moléculaire, à condition que le système catalytique comprenne au moins deux couches de catalyseur constituées d'anneaux de catalyseur, agencées successivement dans la direction d'écoulement du courant gazeux, l'activité des catalyseurs dans des couches de catalyseur adjacentes étant différente les unes des autres, à condition qu'une couche de catalyseur et/ou inerte soit disposée en amont des couches de catalyseurs adjacentes dans la direction opposée à la direction d'écoulement du gaz,
le volume des anneaux inertes et/ou de catalyseur en amont étant inférieur à celui dans au moins une des couches de catalyseur suivantes, ou
le quotient de la surface par volume des anneaux inertes et/ou de catalyseur en amont étant supérieur à celui dans au moins une des couches de catalyseur suivantes et le volume étant réduit par des encoches sur chaque face des anneaux.
